# NEW EUROPEAN PATENT SPECIFICATION

(11) **EP 0 213 800 B2**
(45) Date of publication and mention of the opposition decision: **21.08.1996**
(45) Mention of the grant of the patent: 10.04.1991
(21) Application number: 86306077.8
(22) Date of filing: 06.08.1986
(51) Int. Cl.: C07C 41/16, C07C 43/15

(54) **Monomer Production**
Herstellung eines Monomers
Préparation d'un monomère

(30) Priority: 12.08.1985 GB 8520218; 14.02.1986 GB 8603656
(43) Date of publication of application: 11.03.1987
(73) Proprietor: ALLIED COLLOIDS LIMITED, Bradford, West Yorkshire BD12 0JZ (GB)
(72) Inventor: Farrar, David, Bradford West Yorkshire (GB); Hawe, Malcolm, Huddersfield West Yorkshire (GB)
(74) Representative: Lawrence, Peter Robin Broughton

(56) References cited:
- EP-A- 0 066 179
- FR-A- 1 120 963
- GB-A- 1 273 552
- US-A- 4 142 042
- Methoden der organischen Chemie, Houben-Weyl, VII/2, Sauerstoffverbindungen I/Teil II, 1963, pp. 13, 14
- Chem. Abs., vol. 31, 1937, 1824
- Enc. of Chemical Technology, 3rd ed., vol. 2, 1978, pp. 8, 9

## Description

The present invention relates to a new synthetic process for producing (meth) allyl ethers useful, in particular, as monomers in the production of polymers such as are disclosed in EP0172025 and EP0172723.

It is known to produce alkyl ethers by the Williamson synthesis in which a metal alkoxide is reacted with an alkyl halide or with dialkyl sulphate. The process has been used to produce ally ethers. A metal alkoxide is generally produced by the reaction of the anhydrous alcohol with an alkalimetal or an alkalimetal hydroxide or hydride. For instance, in GB 1273552 it is stated that an allyl ether of a polyalkyleneoxy compound may be synthesised by reacting the compound with allyl chloride in the presence of sodium or potassium hydroxide or metallic sodium.

One problem with the prior art processes is that the reaction of an alkali metal with an alcohol can be dangerous, in particular because of the production of hydrogen gas in the reaction, and difficult to control. Another problem is that reaction is not suitable for many alcohols and the conventional Williamson synthesis is unsatisfactory for many starting materials. For instance, we have tried to use the process when the alcohol is an ethoxylate of stearyl alcohol, but have found that there is little reaction of sodium metal even when the alcohol is molten and shearing is applied to the reaction mixture, such a reaction being dangerous as well as inefficient. When the reaction product is reacted with ally chloride the yield of the desired allyl ether is very low and the product mixture contains a large number of products of undesirable side reactions. Another problem with prior art processes is that when the reaction is carried out in the presence of an alkali metal hydroxide very low yields are obtained.

Another problem is that it is particularly difficult to obtain good yields when the ether contains a polyalkoxy chain. Finally, a serious difficulty with the Production of allyl ether compounds is that the degree of conversion to the desired ether is difficult or impossible to assess without polymerising the product and testing the polymer to check the presence of side-chains derived from the desired ally ether product.

It is known from EP-A-066,179 to make certain polyoxyalkylene mono-allyl or -methyallyl ethers by stoichiometric polymerisation of alkylene oxide with alkali alcoholate of general formula MOR¹ where M is alkali metal and R¹ is C 1 - 4 alkyl, aryl or alkaryl, followed by reaction with allyl halide or methylallyl halide. The reaction is conducted in inert solvent in the absence of moisture from the air, optionally under pressure. The alkylene oxide is stored under pressure.

In US-A-4,142,042 an alkenyl ether is formed from high molecular weight polyol in a liquid phase system containing concentrated alkali metal hydroxide, water, water immiscible hydrocarbon solvent and high molecular weight polyol. In the examples the polyol is reacted with sodium hydroxide and water, some water is removed by azeotropic distillation, allyl chloride is added and further water is removed by azeotropic distillation.

The process according to the invention comprises making a compound of the formula CH₂=CR²CH₂O(C₂H₄O)_{y}R¹ (1) wherein y is an integer of at least 5, R¹ contain 8 to 30 carbon atoms and is selected from alkyl and alkaryl groups and R² is hydrogen, and comprises mixing an alcohol of the formula HO(C₂H₄O)_{y}R¹ wherein R¹ and y are as defined above with a non-reactive solvent having a boiling point of below 150°C and a polarity Eo of up to 0.5 on the Hildebrand scale and selected from hexane, cyclohexane, heptane, petroleum ether, benzene, toluene and xylene, dehydrating the resultant mixture by azeotropic distillation until it is substantially anhydrous and contains below 0.1% by weight water, adding alkali metal alkoxide MOR³ in which M is an alkali metal and R³ is C₁₋₆ alkyl to form the alkali metal alcoholate MO(C₂H₄O)_{y}R¹ wherein R¹ and y are as defined above and an alcohol R³OH, substantially completely removing the alcohol by distillation, and reacting the alcoholate with allyl chloride.

In the process it is necessary to include the initial dehydration step to remove substantially all the water. Water may be present in the starting material or in the reaction vessel. Preferably the amount of water in the reaction mixture is less than 0.05% by weight. We believe that water in the reaction mixture may react with other components of the reaction mixture to give undesirable by-products. For example water may react with the alkali metal alkoxide to form the hydroxide and the etherification reaction would not proceed. Alternatively water may react with the allyl chloride to give the alcohol which would not react in the etherification step; because of the relatively high molecular weight of the starting alcohol even a small amount of water would render much of the starting material inactive.

Although it might be considered possible to remove water from the starting material, by using chemical dehydrating agents, we have found that this is not practical. To enable the water to be removed under conditions which are not such as to form other impurities, it has been found that the water must be removed by azeotropic distillation. It is preferred that the temperature in the reaction vessel is maintained below 130°C or 120°C throughout the process, to reduce side-reactions.

In the new process the solvent is capable of forming an azeotrope with water, and conveniently also with the alcohol R³OH. If the solvent is capable of forming an azeotrope with R³OH it is possible and advantageous to remove that alcohol from the reaction mixture before addition of the halide by azeotropic distillation. If the solvent is not capable of forming an azeotrope with R³OH then it must have a higher boiling point than R³OH in order that the alcohol may be removed from the reaction mixture by distillation ahead of the solvent. However the solvent must have a boiling point of less than 150°C, preferably less than 130°C or 120°C in order that the solvent may be removed from the final product mixture at a temperature at which the allyl ether does not polymerise.

If R³OH is not substantially entirely removed from the reaction mixture before addition of allyl chloride then poor yields of the desired product are obtained.

The solvent may also act to remove volatile by-products or unreacted starting materials from the product mixture.

The solvent is unreactive in the reaction mixture. The solvent may be non-polar; for best results, however, it is preferred that the solvent has some degree of polarity, for example, a polarity Eo of up to 0.5 preferably up to 0.3 on the Hildebrand scale although it should be substantially immiscible with water. The solvent is selected from hexane, cyclohexane, heptane, petroleum ether fractions, benzene, xylene and toluene. It is economic to recycle the solvent distilled over during or at the end of the process and so it is advantageous for the solvent to consist of a single component. The preferred solvent is toluene.

In conventional Williamson syntheses the reaction mixtures are homogeneous, any solid reactants being soluble in the liquid reactants or in their carrier solvents. Thus, for example in a reaction using sodium methoxide in methanol reacting initially with benzyl alcohol, the sodium methoxide is freely soluble in the benzyl alcohol/methanol mixture. There has thus never been any need for the inclusion of other solvents. Although it is not expected that alkali metalalkoxides would be insufficiently soluble in a mixture of the corresponding alcohol, used as a carrier solvent, and the starting alcohol, we have found that the starting alcohol will not react to a sufficient degree with sodium alkoxide in the absence of a solvent of the specified type.

At the end of the reaction the solvent is generally removed from the product allyl ether. It may be removed by evaporation, and it is generally found convenient to lower the temperature at which it may be removed to minimise breakdown of the allyl ether product. This may be done, for example by adding a cosolvent with which the solvent may form an azeotropic mixture and azeotroping the two solvents. A suitable cosolvent has been found to be water. Alternatively or additionally, the pressure above the product mixture is reduced to lower the temperature at which the solvent is removed. Although the alkali-metal halide may be removed from the allyl ether product if desired, it is often unnecessary to do so since it has little adverse effect on the product.

The alkali metal alkoxide is a lower alkoxide having from 1 to 6 carbon atoms, preferably the methoxide or ethoxide. The alkali metal is generally potassium or, preferably, sodium. The alkoxide is for example sodium ethoxide, most preferably sodium methoxide. The alkoxide is generally supplied in the form of a solution, for example in the corresponding alcohol. Thus sodium methoxide is generally supplied in the form of a solution in methanol. Any such solvent is substantially completely removed from the reaction mixture before addition of the allyl chloride, to minimise side reactions. Alcohols may be removed by evaporation, eg fractional distillation, or may form an azeotrope with the reaction solvent.

If one attempts to carry out the reaction using, e.g., sodium metal in place of alkali metal alkoxide (i.e., as in a conventional Williamson synthesis), the reaction proceeds slowly at first as the reaction mixture is inhomogeneous. To try to render the mixture homogeneous, for example by blending is extremely dangerous. The reaction is liable to produce hydrogen as a by-product which is very explosive. Hydrolysis of the halide or some other reaction that prevents the etherification reaction proceeding seems to occur if water is present to assist the formation of a homogeneous mixture.

If alkali metal hydroxide is used in place of the alkoxide (e.g., as suggested in GB 1,275,552) hydrolysis of the halide starting material or some other undesirable interfering side reaction seems to occur and prevents the etherification proceeding. y is often above 10, usually 100 or below 100 and frequently 10 or 15 to 30.

Preferably R¹ comprises 10-24 and most preferably 12-18 carbon atoms. R¹ may be selected from alkyl, for instance octyl, lauryl or stearyl, or, alkaryl such as alkylphenyl, wherein the alkyl group generally contains 6-12 carbon atoms, or mixtures of one or more such groups.

The products of the invention are generally used as monomers in polymerisation reactions. They may be homopolymerised or, preferably, are copolymerised with other comonomers, generally ethylenically unsaturated comonomers generally free of hydrophobic side chains. The polymers may be made by any conventional polymerisation process.

The following example illustrates the invention.

### Example

710g of a 10 mole ethoxylate of stearyl alcohol and 355g xylene are placed in the reaction vessel and dehydrated by azeotroping. 54g sodium methoxide as a 30% solution in methanol is added and the temperature raised to reflux. The methanol produced is removed by distillation. When all the methanol has been removed the reaction mixture is cooled to 60°C and 76.5g allyl chloride is added carefully. The mixture is then heated to 110°C for one hour. After one hour the mixture is transferred to a rotary evaporator where the xylene is removed under 760 mm Hg vacuum and 100°C. Once the xylene is removed the finished product is left to cool.

The example was repeated using a range of different solvents for the reaction components. The quality of the allyl ether monomer produced was assessed by polymerising with ethyl acrylate and methacrylic acid monomers in the presence of varying amounts of a mercapto chain transfer agent and testing the viscosity properties of the resultant polymer. By this means the presence of ethoxylated alkyl side chains in the polymer and of crosslinking due to undesirable by-products containing two or more ethylenically unsaturated groups could be detected. The monomer quality was rated on a scale of 0-10, 10 being very good.

The following table shows the results for each solvent.

**Table**

| Solvent | Monomer Quality |
|---|---|
| Toluene | 9 |
| Cyclohexane | 5 |
| Petroleum ether 60/80 | 5 |
| Petroleum ether 81/100 | 9 |
| Petroleum ether 100/120 | 9 |
| Heptane | 8 |
| Hexane | 7 |

## Claims

1. A process of making a compound of the formula CH₂ = CR²CH₂O(C₂H₄O)_{y}R¹ (1) wherein y is an integer of at least 5, R¹ contain 8 to 30 carbon atoms and is selected from alkyl and alkaryl groups and R² is hydrogen, the process comprising mixing an alcohol of the formula HO(C₂H₄O)_{y}R¹ wherein R¹ and y are as defined above with a non-reactive solvent having a boiling point of below 150°C and a polarity Eo of up to 0.5 on the Hildebrand scale and selected from hexane, cyclohexane, heptane, petroleum ether, benzene, toluene and xylene, dehydrating the resultant mixture by azeotropic distillation until it is substantially anhydrous and contains below 0.1% by weight water, adding an alkali metal alkoxide MOR³ in which M is an alkali metal and R³ is C₁₋₆ alkyl to form the alkali metal alcoholate MO(C₂H₄O)_{y}R¹ wherein R¹ and y are as defined above and an alcohol R³OH, substantially completely removing the alcohol by distillation, and reacting the alcoholate with allyl chloride.

2. A process according to claim 1 in which MOR³ is sodium methoxide.

3. A process according to claim 1 or claim 2 conducted at a temperature below 130°C.

4. A process according to any preceding claim in which the said solvent is separated from the product of formula 1 and is recycled in the process.

5. A process according to any preceding claim in which the solvent is toluene.

## Patentansprüche

1. Verfahren zur Herstellung einer Verbindung der Formel CH₂=CR²CH₂O(C₂H₄O)_{y}R¹ (1), worin y eine ganze Zahl von mindestens 5 ist, R¹ 8 bis 30 Kohlenstoffatome enthält und aus Alkyl- und Alkarylgruppen ausgewählt ist, und R² Wasserstoff ist; welches Verfahren umfaßt das Mischen eines Alkohols der Formel HO(C₂H₄O)_{y}R¹, worin R¹ und y wie oben definiert sind, mit einem nicht-reaktiven Lösungsmittel mit einem Siedepunkt unter 150°C und einer Polarität Eo von bis zu 0,5 auf der Hildebrand-Skala und aus Hexan, Cyclohexan, Heptan, Petrolether, Benzol, Toluol und Xylol ausgewählt, Dehydratisieren der resultierenden Mischung durch azeotrope Destillation, bis sie im wesentlichen wasserfrei ist und weniger als 0,1 Gew.-% Wasser enthält, Zugeben eines Alkalimetallalkoxids MOR³, worin M ein Alkalimetall darstellt und R³ für C₁₋₆-Alkyl steht, um das Alkalimetallalkoholat MO(C₂H₄O)_{y}R¹, worin R¹ und y wie oben definiert sind, und einen Alkohol R³OH zu bilden, im wesentlichen vollständiges Entfernen des Alkohols durch Destillation und Umsetzen des Alkoholats mit Allylchlorid.

2. Verfahren nach Anspruch 1, worin MOR³ Natriummethoxid ist.

3. Verfahren nach Anspruch 1 oder Anspruch 2, durchgeführt bei einer Temperatur unter 130°C.

4. Verfahren nach irgendeinem der vorhergehenden Ansprüche, worin das Lösungsmittel vom Produkt der Formel 1 abgetrennt und wieder in das Verfahren zurückgeführt wird.

5. Verfahren nach irgendeinem der vorhergehenden Ansprüche, worin das Lösungsmittel Toluol ist.

## Revendications

1. Procédé pour préparer un composé de formule CH₂=CR²CH₂O(C₂H₄O)_{y}R¹(1), dans laquelle y est un entier au moins égal à 5, R¹ est choisi parmi les groupes alkyles et alkylaryles en C₈-C₃₀ et R² et un atome d'hydrogène, le procédé comprenant le mélange d'un alcool de formule HO(C₂H₄O)_{y}R¹, dans laquelle R¹ et y sont définis comme ci-dessus, avec un solvant inerte ayant un point d'ébullition de moins de 150°C et une polarité Eo allant jusqu'à 0,5 sur l'échelle de Hildebrand et choisi parmi l'hexane, le cyclohexane, l'heptane, l'éther de pétrole, le benzène, le toluène et le xylène, la déshydratation du mélange résultant par distillation azéotropique jusqu'à ce qu'il soit pratiquement anhydre et contienne moins de 0,1 % en poids d'eau, l'ajout d'un alkylate de métal alcalin MOR³, dans laquelle M est un métal alcalin et R³ est un groupe alkyle en C₁-C₆, pour former l'alcoolate de métal alcalin MO(C₂H₄O)_{y}R¹, dans laquelle R¹ et y sont définis comme ci-dessus, et d'un alcool R³OH, la séparation à peu près complète de l'alcool par distillation et la réaction de l'alcoolate avec du chlorure d'allyle.

2. Procédé selon la revendication 1, dans lequel MOR³ est le méthylate de sodium.

3. Procédé selon la revendication 1 ou 2, mis en oeuvre à une température de moins de 130°C.

4. Procédé selon l'une quelconque des revendications précédentes, dans lequel ledit solvant est séparé du produit de formule 1 et recyclé dans le procédé.

5. Procédé selon l'une quelconque des revendications précédentes, dans lequel le solvant est le toluène.
